# EUROPEAN PATENT SPECIFICATION

(11) **EP 2 914 237 B1**
(45) Date of publication and mention of the grant of the patent: **01.11.2017**
(21) Application number: 13763235.2
(22) Date of filing: 16.09.2013
(51) Int. Cl.: A61K 8/365, A61Q 5/04, A61Q 5/08, A61K 8/19, A61K 8/22

(54) **PROCESS FOR STRAIGHTENING AND BLEACHING HAIR**
VERFAHREN ZUM GLÄTTEN UND BLEICHEN DER HAARE
PROCÉDÉ PERMETTANT DE LISSER ET DE DÉCOLORER DES CHEVEUX

(30) Priority: 05.11.2012 EP 12191339
(43) Date of publication of application: 09.09.2015
(73) Proprietor: Kao Corporation, Chuo-ku Tokyo 103-8210 (JP)
(72) Inventor: ROSE, Burkhard, 64297 Darmstadt (DE); WOOD, Jonathan, 69469 Weinheim (DE)
(74) Representative: Hoffmann Eitle
(86) International application number: PCT/EP2013/069135
(87) International publication number: WO 2014/067703

(56) References cited:
- EP-A1- 2 123 250
- EP-A1- 2 468 247
- WO-A1-2011/105410
- WO-A1-2012/105985
- WO-A2-2011/104282
- WO-A2-2011/139433
- US-A- 3 218 234
- US-A- 4 148 329
- US-A- 4 275 748

## Description

The present invention relates to a method for semipermanent straightening and bleaching of the hair in two steps.

### Background of the Invention

A known method for straightening curly or frizzy hair involves the use of straightening irons. The high temperature of the iron leads to a breakage of hydrogen bonds in the keratin of the hair, achieving a temporary straightening. The hydrogen bonds are formed again by the action of moisture, so that the hair reverts back to its original shape over the time because of air humidity, and the straightening effect vanishes after washing the hair.

The shape of the hair is largely determined by the disulfide bonds linking two cysteine moieties of the hair keratin. In order to achieve a more permanent shaping of the hair, known methods involve the cleavage of the disulfide bonds by the action of a sulfide- or thio group containing reducing agent. After the hair has been brought into the desired shape, new disulfide bonds are formed by applying an oxidizing agent such as hydrogen peroxide, thus fixing the shape of the hair. The use of such agents, however, may cause damage to the hair.

As an example for this kind of hair shaping treatment, reference is made to GB 1 416 564, describing reducing compositions comprising thioglycolates or thiolactates as reducing agents and fixing compositions comprising hydrogen peroxide as an oxidizing agent. The reducing compositions may further comprise a salt of an acid such as glyoxylic acid as a buffering agent.

As an alternative to the above-described two-step reduction and oxidation process, the disulfide bridges can be cleaved by the action of an alkaline agent such as sodium hydroxide at a pH of about 11 or higher. Under these conditions, the disulfide (or cystin) moiety can undergo a disproportionation reaction under the elimination of sulfur, and is cleaved into an alpha-beta-unsaturated dehydro-alanine moiety and a cysteine moiety. After the hair has been brought into the desired shape, the dehydro-alanin moieties and the cysteine moieties form thioether bonds and combine to lanthionine, stabilizing the straightened state of the hair. Since the disulfide or cysteine moieties are converted into lanthionine moieties, this type of hair straightening process using an alkaline agent is also called lanthionization.

Both the two-stage reduction/oxidation method and the lanthionization method rely on a cleavage of the disulfide bonds and the formation of new bonds among the hair proteins, leading to an irreversible change of the shape of the hair. This means that these processes can achieve a permanent straightening, wherein the treated portion of the hair maintains its shape, and the straightening effect only vanishes because of the growth of the hair.

Recently, it has been found that carboxylic acids having a carbonyl group adjacent to the carboxy group, such as glyoxylic acid, which are known as a buffering agent in cosmetic compositions, may have a semi-permanent straightening effect when used in combination with mechanical straightening means. In this connection, semi-permanent means that the hair maintains its straightened appearance for several washing cycles. This is in contrast to permanent straightening, wherein the shape of the hair is modified irreversibly, as in the above-described methods based on the cleavage of the disulfide bonds, and in contrast to the transient straightening achieved with a straightening iron, which vanishes by the action of moisture.

In this respect, WO 2011/104282 describes a process for semi-permanent hair straightening, which involves applying a composition comprising an α-keto acid onto the hair, leaving the composition in contact with the hair for 15 to 120 minutes, drying the hair and straightening the hair with a straightening iron at a temperature of 200±50°C.

Furthermore, WO 2012/010351 describes a treatment for semi-permanent straightening of curly, frizzy or wavy hair by applying a solution of glyoxylic acid in combination with mechanical straightening, using a straightening iron at a temperature of 200±30°C. After the treatment, the hair is said to retain its shape for at least six consecutive washings.

WO 2012/105985 likewise describes a straightening procedure using glyoxylic acid, wherein the hair may subsequently be treated with an oxidative conditioner.

WO 2011/139433 describes procedures for lightening the color of hair that has been recently contacted with a relaxing or straightening composition. The described procedure involves the application of an alkaline relaxing composition, followed by the application of a lightening composition having a pH from about 2 to about 7. Optionally, the lightening composition may also comprise a dye.

Hair bleaching involves the oxidative decomposition of the melanin pigments in the hair by the action of an oxidizing agent, thus bleaching, blonding or lightening the hair colour. Typically, alkaline hydrogen peroxide or peroxide compounds such as persulfates are used as the oxidizing agent.

The hair bleaching composition is generally of a two-component type, wherein the first component is a water-free composition comprising an alkalizing agent, while the second component is an aqueous hydrogen peroxide solution. The components are mixed prior to the application to the hair.

The water-free first component may be a bleaching powder comprising a persulfate in combination with an alkaline agent. In order to reduce the dustiness, the powder may be agglomerated with a binding agent, yielding a granular composition. A usual binding agent is mineral oil, which is the subject matter of EP 560 088 B1. Furthermore, EP 778 020 A1 suggests the use of oil and wax compounds or their mixtures for preparation of suspensions.

Because of the strong oxidative activity, the hair may be damaged in the bleaching process, specifically in case the hair is repeatedly bleached. EP 2 468 247 addresses the problem of alleviating the risk of hair damage and describes a substantially water-free hair bleaching and/or lightening composition characterized by comprising a diamide compound.

The combination of these straightening and hair bleaching methods, however, has not yet been described in the state of the art.

### Summary of the Invention

The present invention relates to a process for bleaching and straightening hair, which comprises the following steps performed in this order:
(a) application of a first composition (Straightening composition) having a pH of 4 or lower and comprising glyoxylic acid and/or a hydrate thereof and/or a salt thereof onto the hair;
(b) leaving the composition on the hair for 1 to 120 minutes,
(c) drying the hair,
(d) optionally rinsing off the hair with water and drying the hair,
(e) treating the hair with an iron having a surface temperature of 180 ± 50°C,
(f) rinsing the hair and optionally shampooing and drying the hair,
(g) application of an aqueous second composition (bleaching composition) comprising at least one oxidizing agent and at least one alkalizing agent and having a pH of 8 to 11 onto the hair,
(h) leaving the composition on the hair for 5 to 45 minutes, rinsing off the composition and optionally shampooing and drying the hair,
wherein steps (g) to (h) are carried out directly after steps (a) to (f).

In this connection, "directly after" means that the time interval between the completion of steps (a) to (f) and the beginning of steps (g) to (h) is not more than two or three hours, preferably not more than 1 hour, more preferably not more than 30 minutes.

The process of the invention achieves a semi-permanent straightening of the hair. Generally, hair straightening may be divided into two main categories. The first is permanent straightening, wherein hair shape is irreversibly changed either by a two-step reduction and oxidation process or alternatively by treating hair with a strong alkaline composition having pH values 11.0 or above.

The second is semi-permanent straightening wherein hair is straightened relatively reversibly and returns to its approximately original shape after a certain number of washing cycles. The present invention relates to semi-permanent straightening, and does not involve the cleavage of disulfide bonds by sulfur-based reducing agents or alkali.

In another aspect, the present invention relates to a hair treatment kit for carrying out the above process, comprising the straightening composition and the bleaching composition as defined above for use in the above-described process.

### Detailed Description of the Invention

Conventionally, straightening and bleaching treatment of the hair is carried out in two separate sessions, which is time consuming and uneconomical for end users.

Besides, hair bleaching usually causes hair damage because of the action of the oxidizing agent. In turn, this leads to inferior mechanical properties of the hair, and may also result in a dull appearance.

Such hair damage is also known to be caused by conventional hair straightening techniques, e.g., by the action of the disulfide-cleaving agents and/or the agents for forming new disulfide bonds. Accordingly, the combination of conventional hair straightening directly before hair bleaching was likely to cause severe hair damage, resulting in dull appearance and poor mechanical properties of the hair.

The present invention solves this problem by providing a method which achieves a straightening and bleaching of the hair in two steps in immediate succession in a single salon visit and simultaneously protects the hair from damage.

For this purpose, the method of the present invention uses a first composition (straightening composition) comprising glyoxylic acid and a second composition (bleaching composition) comprising least one oxidizing agent and at least one alkalizing agent and having a pH of 8 to 11. The hair is treated with the said compositions successively, so that the bleaching treatment is performed directly after the straightening treatment.

With the term "directly after", it is meant that the time interval between the two treatments is not more than two or three hours, preferably not more than one hour, more preferably is not more than 30 minutes.

Surprisingly, it has been found that the straightening treatment using glyoxylic acid and the bleaching treatment can be performed in direct succession without any adverse interactions.

On the contrary, the hair treatment method of the present invention unexpectedly yields improvements of the mechanical properties of the hair, e.g., in comparison to the case where the bleaching is performed alone. The mechanical properties may, be measured by the stress-strain method.

### 1. The Straightening Composition

The straightening composition comprises glyoxylic acid as the active component.

The glyoxylic acid may be comprised in the composition in its free acid form. The carbonyl group adjacent to the acid group of the acid may also be present in the hydrate form. Apart from the free acid form and the hydrate thereof, salts of the acid or the hydrate may also be used.

The hydrate may be formed when providing the composition as an aqueous solution. Glyoxylic acid (H-CO-COOH) in aqueous solution is almost quantitatively present as the hydrate (H-C(OH)₂-COOH). Besides, the hydrate may also condense to dimers.

A salt of glyoxylic acid may also be used. As examples, alkali metal salts such as the sodium or potassium salt, alkaline earth metal salts such as the magnesium salt or the calcium salt may be mentioned.

The concentration of the glyoxylic acid and/or a hydrate thereof and/or salts thereof is in the range of 0.1 to 40%, preferably 0.5 to 30%, more preferably 1 to 25% and even more preferably 2.5 to 20% by weight, based on the total weight of the straightening composition.

The pH of the straightening composition is below or equal to 4.0, preferably in the range of 0.5 to 3, more preferably 1 to 2.5, as measured directly and at ambient temperature (25°C). The pH of the compositions may be adjusted using known alkaline solutions, preferably with sodium hydroxide solution.

As discussed above, conventional permanent hair shaping/straightening techniques are based on the reorganization of the disulfide bridges and involve a cleavage of the disulfide bonds either by using a sulfur-based reducing agent or an alkali agent, followed by the shaping of the hair and the formation of new bonds (i.e., disulfide bonds formed by the action of an oxidizing agent or thioether bonds, respectively). In contrast to these permanent straightening methods, the present invention does not utilize cleavage of the disulfide bonds and fixing the bonds in the new shape. Therefore, the straightening composition of the present invention does not require the presence of sulfur-based reducing agents. However, up to 2% by weight calculated to the total of the composition sulfur based reducing agents does not disturb the straightening performance of the compositions. Therefore, the straightening composition has less than 2% by weight of sulfur-based reducing agents, and preferably is free of sulfur-based reducing agents.

The straightening composition may suitably comprise further ingredients such as surfactants and/or conditioning component as defined below, and may suitably be in the form of a solution, emulsion, cream, paste and mousse.

### 2. Surfactant

The straightening composition may comprise a surfactant. As the surfactant, any of a cationic surfactant, a nonionic surfactant, an amphoteric surfactant and an anionic surfactant can be used. It is also possible to use two or more types of surfactants in combination.

The cationic surfactant is preferably a mono-long chain alkyl quaternary ammonium salt, having a C₈-C₂₄ alkyl residue and three C₁-C₄ alkyl residues.

Preferably at least one mono alkyl quaternary ammonium surfactant is selected from the compounds with the general formula 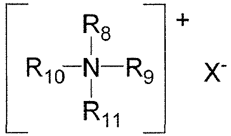 wherein R₈ is a saturated or unsaturated, branched or straight alkyl chain with 8-22 C atoms or

R₁₂-CO-NH-(CH₂)ₙ-

wherein R₁₂ is a saturated or unsaturated, branched or straight alkyl chain with 7-21 C atoms and n is an integer of 1 - 4, or

R₁₂-CO-O-(CH₂)ₙ-

wherein R₁₂ is a saturated or unsaturated, branched or straight alkyl chain with 7-21 C atoms and n is an integer of 1 - 4, and
R₉, R₁₀ and R₁₁ are independent from each other an alkyl group with 1 to 4 carbon atoms, hydroxyl alky chain with 1 to 4 carbon atoms, or ethoxy or propoxy group with a number of ethoxy or propoxy groups varying in the range of 1 to 4, and X is chloride, bromide, methosulfate or ethosulfate.

Suitable cationic surfactants are, for example, long-chain quaternary ammonium compounds which can be used alone or in admixture with one another, such as cetyl trimethyl ammonium chloride, myristyl trimethyl ammonium chloride, behentrimonium chloride, trimethyl cetyl ammonium bromide, stearyl trimethyl ammonium chloride, stearyl trimonium chloride and stearamidopropyltrimonium chloride.

Examples of the nonionic surfactant include polyoxy-C₁₋₄-alkylene C₈₋₂₄-alkyl ether, polyoxy-C₁₋₄-alkylene C₈₋₂₄-alkylene alkenyl ether, higher (C₁₂-C₂₄) fatty acid sucrose ester, polyglycerin C₈₋₂₄-fatty acid ester, higher (C₁₂-C₂₄) fatty acid mono- or diethanolamide, polyoxyethylene hardened castor oil, polyoxyethylene sorbitan C₈₋₂₄-fatty acid ester, polyoxyethylene sorbit C₈₋₂₄-fatty acid ester, C₈₋₂₄-alkyl saccharide surfactant, C₈₋₂₄-alkylamine oxide, and C₈₋₂₄-alkylamidoamine oxide.

Examples of the amphoteric surfactant include an imidazoline-based surfactant, a carbobetaine-based surfactant, an amidobetaine-based surfactant, a sulfobetaine-based surfactant, a hydroxysulfobetaine-based surfactant and an amidosulfobetaine-based surfactant.

Examples of the anionic surfactant include alkylbenzenesulfonate, alkyl or alkenyl ether sulfate, alkyl or alkenyl sulfate, olefin sulfonate, alkanesulfonate, saturated or unsaturated fatty acid salts, alkyl or alkenyl ether carboxylate, α-sulfo fatty acid salts, N-acylamino acid type surfactants, phosphoric acid mono- or diester type surfactants, and sulfosuccinate. Examples of the alkyl ether sulfate include polyoxyethylene alkyl ether sulfate. Examples of the counterion for the anionic residues of these surfactants include an alkalimetal ion such as sodium ion or potassium ion; an alkaline earth metal ion such as calcium ion or magnesium ion; an ammonium ion; and an alkanolamine having 1 to 3 alkanol groups each having 2 or 3 carbon atoms (for example, monoethanolamine, diethanolamine, triethanolamine, or triisopropanolamine).

The surfactant can be used singly or in combination of two or more kinds. When adding a surfactant to the straightening composition, the content thereof usually is 0.05 to 10% wt.%, more preferably 0.1 to 5 wt.%, based on the total weight of the straightening composition.

### 3. Conditioning Component

The straightening composition may optionally comprise a conditioning component suitable for application to the hair. The conditioning component is an oil or polymer which adheres to the hair and improves the feel and the manageability.

When using the conditioning component, the total amount thereof is preferably 0.01 to 30 wt.%, more preferably 0.05 to 20 wt.%, and even more preferably 0.1% to 10 wt.%, based on the total weight of the straightening composition.

Examples of the conditioning component generally include silicones, higher alcohols, and organic conditioning oils (for example, hydrocarbon oil, polyolefin and fatty acid ester). The composition may comprise a single type of conditioning component, or two or more in combination.

### Silicones

In order to improve the feel of use, the straightening composition preferably contains a silicone. Examples of the silicone include dimethylpolysiloxane, and modified silicone (for example, amino-modified silicone, fluorine-modified silicone, alcohol-modified silicone, polyether-modified silicone, epoxy-modified silicone, or alkyl-modified silicone), but dimethylpolysiloxane, polyether-modified silicone and amino-modified silicone are preferred.

The dimethylpolysiloxane may be any cyclic or non-cyclic dimethylsiloxane polymer, and examples thereof include SH200 series, BY22-019, BY22-020, BY11-026, B22-029, BY22-034, BY22-050A, BY22-055, BY22-060, BY22-083, FZ-4188 (all by Dow Corning Toray Co., Ltd.), KF-9008, KM-900 series, MK-15H, and MK-88 (all by Shin-Etsu Chemical Co., Ltd.).

The polyether-modified silicone may be any silicone having a polyoxyalkylene group, and the group constituting the polyoxyalkylene group may be an oxyethylene group or an oxypropylene group. More specific examples include KF-6015, KF-945A, KF-6005, KF-6009, KF-6013, KF-6019, KF-6029, KF-6017, KF-6043, KF-353A, KF-354A, KF-355A (all by Shin-Etsu Chemical Co., Ltd.), FZ-2404, SS-2805, FZ-2411, FZ-2412, SH3771M, SH3772M, SH3773M, SH3775M, SH3749, SS-280X series, BY22-008 M, BY11-030, and BY25-337 (all by Dow Corning Toray Co., Ltd.).

The amino-modified silicone may be any silicone having an amino group or an ammonium group, and examples thereof include an amino-modified silicone oil having all or a part of the terminal hydroxyl groups capped with a methyl group or the like, and an amodimethicone which does not have the terminals capped. A preferred example of the amino-modified silicone may be a compound represented by the following formula: 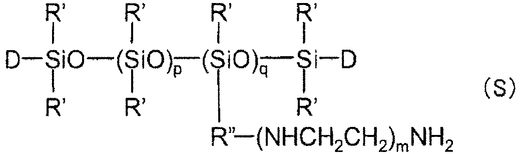 wherein R' represents a hydroxyl group, a hydrogen atom or R^{X}; R^{X} represents a substituted or unsubstituted monovalent hydrocarbon group having 1 to 20 carbon atoms; D represents R^{X}, R"-(NHCH₂CH₂)ₘNH₂, OR^{X}, or a hydroxyl group; R" represents a divalent hydrocarbon group having 1 to 8 carbon atoms; m represents a number from 0 to 3; p and q represent numbers, the sum of which is, as a number average, equal to or greater than 10 and less than 20,000, preferably equal to or greater than 20 and less than 3000, more preferably equal to or greater than 30 and less than 1000, and even more preferably equal to or greater than 40 and less than 800.

Specific examples of suitable commercially available products of the amino-modified silicone include amino-modified silicone oils such as SF8452C, SS-3551 (all by Dow Corning Toray Co., Ltd.), KF-8004, KF-867S, and KF-8015 (all by Shin-Etsu Chemical Co. , Ltd.); and amodimethicone emulsions such as SM8704C, SM8904, BY22-079, FZ-4671, and FZ-4672 (all by Dow corning Toray Co., Ltd.).

The total content of these silicones in the composition of the present invention is usually 0.1 to 20 wt.%, preferably 0.2% to 10 wt.% and more preferably 0.5 to 5 wt.%, based on the total weight of the straightening composition.

### Oil component

For improving the feel upon use, the straightening composition may also include an organic conditioning oil. The organic conditioning oil that is suitably used as a conditioning component is preferably a low-viscosity and water-insoluble liquid, and is selected from a hydrocarbon oil having at least 10 carbon atoms, a polyolefin, a fatty acid ester, a fatty acid amide, a polyalkylene glycol, and mixtures thereof. The viscosity of such an organic conditioning oil as measured at 40°C is preferably 1 to 200 mPa·s, more preferably 1 to 100 mPa·s, and even more preferably 2 to 50 mPa·s. The viscosity may be determined using a capillary viscometer.

Examples of the hydrocarbon oil include a cyclic hydrocarbon, a linear aliphatic hydrocarbon (saturated or unsaturated), and a branched aliphatic hydrocarbon (saturated or unsaturated), and polymers or mixtures thereof are also included. The linear hydrocarbon oil preferably has 12 to 19 carbon atoms. The branched hydrocarbon oil includes hydrocarbon polymers, and preferably has more than 19 carbon atoms.

The polyolefin is a liquid polyolefin, more preferably a liquid poly-α-olefin, and even more preferably a hydrogenated liquid poly-α-olefin. The polyolefin used herein is prepared by polymerizing an olefin monomer having 4 to 14 carbon atoms, and preferably 6 to 12 carbon atoms.

The fatty acid ester may be, for example, a fatty acid ester having at least 10 carbon atoms. Examples of such a fatty acid ester include esters having a hydrocarbon chain derived from a fatty acid and an alcohol (for example, monoesters, polyhydric alcohol esters, or di- and tricarboxylic acid esters). The hydrocarbon group of these fatty acid esters may have another compatible functional group such as an amide group or an alkoxy group as a substituent, or the hydrocarbon group may be covalently bonded to those functional groups. More specifically, an alkyl and alkenyl ester of a fatty acid having a fatty acid chain having 10 to 22 carbon atoms, a carboxylic acid ester of an aliphatic alcohol having an aliphatic chain derived from an alkyl and/or alkenyl alcohol having 10 to 22 carbon atoms, and a mixture thereof are suitably used. Specific examples of these preferred fatty acid esters include isopropyl isostearate, hexyl laurate, isohexyl laurate, isohexyl palmitate, isopropyl palmitate, decyl oleate, isodecyl oleate, hexadecyl stearate, decyl stearate, dihexadecyl adipate, lauryl lactate, myristyl lactate, cetyl lactate, oleyl stearate, oleyl oleate, oleyl myristate, lauryl acetate, cetyl propionate and dioleyl adipate.

Further suitable oil components are natural oils such as paraffin oil and natural triglycerides.

Suitable natural triglycerides are argan oil, shea butter oil, karite oil, olive oil, almond oil, avocado oil, ricinus oil, coconut oil, palm oil, sesame oil, peanut oil, sunflower oil, peach kernel oil, wheat germ oil, macadamia nut oil, macadamia oil, night primrose oil, jojoba oil, castor oil, soya oil, lanolin, passiflora oil, black cumin oil, borage oils, grapeseed oil, hempseed oil, kukui nut oil, and rosehip oil.

The organic conditioning oil may be used in combination of two or more kinds, and the total concentration is typically in the range of 0.1 to 20 wt.%, preferably 0.2 to 10 wt.%, more preferably 0.5 to 5 wt.%, based on the total weight of the straightening composition.

### Alcohols

From the viewpoint of improving the sense of touch and stability, the hair straightening composition may also contain a higher alcohol having 8 carbon atoms or more. Usually, the higher alcohol has 8 to 22 carbon atoms, and preferably 16 to 22 carbon atoms. Specific examples thereof include cetyl alcohol, stearyl alcohol, behenyl alcohol, and mixtures thereof.

The higher alcohol may be used in combination of two or more kinds, and the content thereof is typically 0.1 to 20 wt.%, preferably 0.2 to 10 wt.%, more preferably 0.5 to 5 wt.%, based on the total weight of the straightening composition.

Additionally polyols may suitably be comprised in the composition. Examples of the polyalkylene glycol include polyethylene glycol and polypropylene glycol, and a mixture of the two may be used, or a copolymer of ethylene oxide and propylene oxide may also be used.

### 4. The Bleaching Composition

The bleaching, blonding or lightening of the hair colour is achieved by applying a bleaching composition which causes the oxidative decomposition of the melanin pigments in the hair. The bleaching composition comprises at least one oxidizing agent and at least one alkalizing agent. Typically, alkaline hydrogen peroxide or peroxide compounds such as persulfates are used as the oxidizing agent in an aqueous medium.

There are no particular limitations with respect to the bleaching composition used in the process of the present invention. Any conventional bleaching, blonding or lightening composition may be used.

The concentration of hydrogen peroxide in the bleaching composition is usually within the range of 1 to 20 wt.%, preferably 1 to 15 wt.%, more preferably 2 to 12 wt.%, based on the total weight of the composition, depending on the desired degree of bleaching. In order to increase the bleaching effect, hydrogen peroxide and other peroxide compounds may be used in combination. The bleaching composition has an alkaline pH of 8 to 11, in particular 9 to 10.

Typically, the bleaching composition comprises at least two components, which are stored separately and mixed prior to application onto the hair. The first component comprises an alkalizing agent and the second component is an aqueous hydrogen peroxide composition, wherein the two components are stored separately and mixed prior to application onto the hair. Examples of the alkalizing agent in the first component include ammonia, mono ethanol amine, isopropanolamine, 2-amino-2-methyl propanol, 2-amino-2-hydroxy-1,3-propanediol and 2-aminobutanol, and a salt thereof; alkanediamine such as 1,3-propanediamine and a salt thereof; and carbonate such as guanidine carbonate, sodium carbonate, potassium carbonate, sodium bicarbonate, and potassium bicarbonate and their mixtures.

The aqueous hydrogen peroxide solution preferably has a concentration of 1 to 20 wt.%, more preferably 2 to 12 wt.%, based on the total weight of the composition. The pH is preferably 2 to 6, more preferably 2.5 to 4. The pH may be adjusted by means of suitable organic and inorganic acids, bases and buffering agents.

The hydrogen peroxide of the second component may be used as the only oxidizing agent. Bleaching compositions of this type are usually termed "lightening compositions". It is possible to use a mixture of an aqueous ammonia solution and an aqueous hydrogen peroxide solution as a lightening composition.

As a preferable example for a lightening composition, an aqueous solution of 3-5 wt.% ammonia and 3-5 wt.% hydrogen peroxide may be mentioned. The aqueous solutions of ammonia and the hydrogen peroxide are mixed directly before use.

For increasing the bleaching effect, however, the first component preferably contains a further oxidizing agent in solid form. As examples for the oxidizing agent, persulfates such as sodium, potassium and ammonium persulfate, earth alkali peroxides such as magnesium peroxide, melamine peroxide or urea peroxide or phthalimide peroxyhexanoic acid, and mixtures thereof may be mentioned. The proportion of the oxidizing agent in the first component is at least 5%, preferably in the range of 20 to 80%, more preferably 25 to 70% and yet more preferably 30 to 60% by weight, based on the total weight of the composition. Bleach powders, such as those described in EP 2 468 247, may be mentioned as examples for this type of compositions.

For a bleaching composition with a stronger bleaching effect, it is preferable to use a bleach powder comprising a persulfate salt such as sodium, potassium and ammonium persulfate, in an amount of 20 to 60 wt.% and an alkali metasilicate such as sodium silicate as a basic component in an amount of 5 to 15 wt.%, based on the total weight of the bleaching powder. The remainder may be a powder base material such as diatomeous earth. The bleach powder is mixed with 4 to 12 wt.% hydrogen peroxide composition at a ratio of preferably 1:1 before use.

### 5. Hair Treatment Method

The hair treatment process of the present invention comprises two stages, namely the straightening treatment (steps (a) to (f)) and the bleaching treatment (steps (g) to (h)).

The straightening treatment achieves a semi-permanent straightening of the hair, utilizing glyoxylic acid as the active agent. The straightening effect is not achieved by cleaving the disulfide bonds by reduction or the action of strong alkali. Accordingly, the usage of a reducing composition or an alkaline relaxer (lanthionization agent) is not required.

In step (a), the straightening composition is applied to the hair. The application weight ratio of hair to composition is preferably from 0.5:2 to 2:0.5, preferably 0.5:1 to 1:0.5, more preferably about 1:1.

Subsequent to the application, the hair straightening composition is left on the hair for 1 to 120 minutes, preferably 5 to 90 minutes, more preferably 10 to 60 minutes, and in particular 15 to 45 minutes at a temperature range of 20 to 45°C and preferably at ambient temperature (step (b)). The time the composition is left on the hair depends on the type of the hair, on whether the hair is damaged and/or resistant.

After the processing, the hair is dried in order to avoid an excessive steam generation in the subsequent step of treating the hair with the iron (step (c)). Typically, a hair dryer is used for this purpose. It is preferable to dry the hair under continuous combing in order to prevent entanglement of the hair.

Optionally, the hair may be rinsed off with water (step (d)) and dried.

Subsequent to the drying, the hair is treated with an iron (step (e)). A usual straightening iron may be used for this purpose. In order to prevent damage to the hair, the surface temperature of the iron is 250°C or lower, preferably 230°C or lower, more preferably 200°C or lower. For achieving a good straightening effect, the temperature of the iron is 130°C or higher, preferably 150°C or higher, more preferably 170° or higher. The iron may preferably have a surface temperature of 180±50°C, more preferably 170 to 200°C.

After the straightening steps, the hair is rinsed and optionally shampooed and dried (step (f)).

Then, the bleaching composition is applied to the hair (step (g)) and allowed to act for 5 to 45 minutes before it is rinsed off (step (h)). In order to achieve an even bleaching or lightening, the time is 5 minutes or longer, preferably 10 minutes or longer. In general, a maximum time of 30 minutes is usually sufficient.

The application ratio of hair to composition by weight is preferably 0.5:2 to 2:0.5, more preferably 0.5:1 to 1:0.5, even more preferably about 1:1.

Then, the composition is rinsed off with water. Optionally, the hair may be shampooed after the rinsing and dried, e.g., by using a hair dryer.

In the present invention, the bleaching steps (g) to (h) are performed directly after the straightening steps (a) to (f), so that the method of the invention can be carried out in a single salon visit.

In this connection, "directly after" means that the interval between the completion of steps (a) to (f) and the beginning of steps (g) to (h) is not more than two or three hours, preferably not more than 1 hour, more preferably not more than 30 minutes.

### Examples

The following examples are to illustrate the invention but not to limit.

### Example 1:

**Lightening composition**

| | % by weight |
|---|---|
| H2O2 | 4% |
| Ammonia | 4% |
| Water | to 100 |

**Straightening composition**

| | % by weight |
|---|---|
| Glyoxylic acid | 10% |
| Amodimethicone | 1% |
| Cetearyl alcohol | 5% |
| Ceteareth-20 | 2% |
| Sodium hydroxide | q.s. to pH 2.0 |
| Water | to 100 |

### Example 2:

**Bleach powder**

| | % by weight |
|---|---|
| Ammonium persulphate | 45 |
| Sodium silicate | 10 |
| Diatomeous earth | to 100 |

Before application to the hair, the bleach powder is mixed with an aqueous 12% H₂O₂ solution at a weight ratio of 1:1. The straightening composition described in Example 1 is used as the straightening composition.

### Example 3:

**Bleach powder**

| | % by weight |
|---|---|
| Sodium persulphate | 20 |
| Potassium persulphate | 25 |
| Sodium silicate | 10 |
| Paraffin oil | 10 |
| Diatomeous earth | to 100 |

Prior to application to the hair, the bleach powder is mixed with an aqueous 9% H₂O₂ solution at a weight ratio of 1:1. The straightening composition described in Example 1 is used as the straightening composition.

The above compositions of Examples 1-3 are used to straighten and to lighten or bleach the hair. It is observed that the strain stress value of hair fibers lightened or bleached after straightening the hair is similar to that of untreated hair which clearly demonstrates surprisingly recovery of hair mechanical properties.

### Test Example

Tresses of human hair (Chinese, 2g each) were used as the test samples for the evaluation of the mechanical properties by Dynamic Mechanical Analysis (DMA).

Sample (A) was not subjected to any treatment and served as a reference.

Sample (B) (comparative) was bleached without a preceding straightening treatment. For this purpose, the hair tress was placed into a solution comprising 24 wt.% of a 50% hydrogen peroxide solution and 5 wt.% of a 25% ammonia solution, the balance being water. The hair trees was left in the bleaching solution for 30 minutes at room temperature and then was rinsed off with water for 5 minutes and blow dried.

Sample (C) was straightened and bleached according to the invention. An aqueous solution containing 10 wt.% glyoxylic acid was used as the straightening composition. The composition was applied to the hair tress at a ratio of solution to hair of 1.5:1 and left on the hair for 15 minutes at room temperature. Then, the hair was dried with a blow drier and straightened with a flat iron (temperature 180°C, 6 strokes). After the straightening treatment, Sample (C) was subjected to the same bleaching treatment as described above for Sample (B).

Ten hairs of each of Sample (A), (B) and (C) were selected and soaked with distilled water for 30 min at 20°C. Then, upon removal from the water, each hair was mounted in the DMA apparatus and the measurement performed.

DMA measurement parameters were as follows:
- Instrument name: Rheometric Scientific DMTA V
- Sample length: 1cm
- Amplitude: 0.1%
- Frequency: 10Hz
- Temperature: 20°C,

From the measured results for each hair, average values for the elastic modulus E' were calculated for each sample (A), (B) and (C). The following results were obtained:

| Sample | E' (GPa) |
|---|---|
| (A) (Untreated Reference): | 5.41 |
| (B) (Bleaching only): | 4.20 |
| (C) (Straighten + Bleach): | 4.71 |

These results show that in comparison to bleaching alone, the method of the present invention leads to superior mechanical properties of the hair.

## Claims

1. Process for straightening and bleaching the hair, which comprises the following steps performed in this order:
(a) application of a first composition (straightening composition) having a pH of 4 or lower and comprising glyoxylic acid and/or a hydrate thereof and/or a salt thereof onto the hair;
(b) leaving the composition on the hair for 1 to 120 minutes;
(c) drying the hair,
(d) optionally rinsing off the hair with water and drying the hair
(e) treating the hair with an iron having a surface temperature of 130 to 250°C, preferably 180 ± 50°C,
(f) optionally rinsing and/or shampooing and drying the hair,
(g) application of an aqueous second composition (bleaching composition) comprising at least one oxidizing agent and at least one alkalizing agent and having a pH of 8 to 11 onto the hair,
(h) leaving the composition on the hair for 5 to 45 minutes, rinsing off the composition and optionally shampooing and drying the hair,
wherein steps (g) to (h) are carried out directly after steps (a) to (f).

2. The process according to claim 1, wherein the oxidizing agent is hydrogen peroxide, preferably in an amount in the range of 1 to 20 wt.%, based on the total weight of the bleaching composition.

3. The process according to claim 1 or 2, wherein the alkalizing agent is ammonia.

4. The process according to any of the claims 1 to 3, wherein the bleaching composition is a two-component composition formed by mixing a solid first component comprising at least one peroxy-salt and the alkalizing agent and an aqueous second component comprising hydrogen peroxide.

5. The process according to claim 4, wherein the solid first component comprises an ammonium salt.

6. The process according to any of the claims 1 to 5, wherein the straightening composition comprises glyoxylic acid and/or a hydrate thereof and/or a salt thereof at a concentration in the range of 0.1 to 40% by weight, calculated on the basis of the total weight of the straightening composition.

7. The process according to any of the claims 1 to 6, **characterized in that** the straightening composition has a pH of 0.5 to 3.

8. The process according to any of the claims 1 to 7, wherein the straightening composition comprises one or more conditioning components.

9. The process according to claim 8, wherein the conditioning component is a silicone.

10. The process according to any of the claims 1 to 9, wherein the straightening composition comprises less than 2% of sulfur-based reducing agents.

11. The process according to any of the claims 1 to 10, wherein the straightening composition and/or the bleaching composition is applied onto hair in an application weight ratio of hair to composition of 0.5:2 to 2:0.5.

12. The process according to any of the claims 1 to 11, wherein the temperature of the iron is in the range of 170 to 200°C.

13. The process according to any of the claims 1 to 12, wherein the combination with the application of a reducing composition or an alkaline relaxer is excluded.

14. Hair treatment kit for straightening and bleaching hair, **characterized in that** it comprises:
a straightening composition, which has a pH of 4 or lower and comprises glyoxylic acid and/or a hydrate thereof and/or a salt thereof; and
a bleaching composition of the two-component type, which comprises a first component comprising at least one alkalizing agent, and a second component comprising at least one oxidizing agent, wherein the first and the second component are provided to be mixed before application, to give a pH of 8 to 11.

15. Use of the hair treatment kit as defined in claim 14 for straightening and bleaching the hair.

## Patentansprüche

1. Verfahren zum Glätten und Bleichen des Haars, das die folgenden, in dieser Reihenfolge durchgeführten Schritte umfasst:
(a) das Aufbringen einer ersten Zusammensetzung (Glättungszusammensetzung), die einen pH-Wert von 4 oder niedriger aufweist und Glyoxylsäure und/oder ein Hydrat davon und/oder ein Salz davon umfasst, auf das Haar,
(b) das Belassen der Zusammensetzung auf dem Haar für 1 bis 120 Minuten,
(c) das Trocknen des Haars,
(d) optional das Spülen des Haars mit Wasser und das Trocknen des Haars,
(e) das Behandeln des Haars mit einem Eisen mit einer Oberflächentemperatur von 130 bis 250°C, bevorzugt 180 ± 50°C,
(f) optional das Spülen und/oder das Shampoonieren und das Trocknen des Haars,
(g) das Aufbringen einer wässrigen zweiten Zusammensetzung (Bleichzusammensetzung), die mindestens ein Oxidationsmittel und mindestens ein Alkalisierungsmittel umfasst und einen pH-Wert von 8 bis 11 aufweist, auf das Haar,
(h) das Belassen der Zusammensetzung auf dem Haar für 5 bis 45 Minuten, das Abspülen der Zusammensetzung und optional das Shampoonieren und das Trocknen des Haars,
wobei die Schritte (g) bis (h) unmittelbar nach den Schritten (a) bis (f) durchgeführt werden.

2. Verfahren gemäß Anspruch 1, bei dem das Oxidationsmittel Wasserstoffperoxid, bevorzugt in einer Menge im Bereich von 1 bis 20 Gew.%, bezogen auf das Gesamtgewicht der Bleichzusammensetzung, ist.

3. Verfahren gemäß Anspruch 1 oder 2, bei dem das Alkalisierungsmittel Ammoniak ist.

4. Verfahren gemäß einem der Ansprüche 1 bis 3, bei dem die Bleichzusammensetzung eine Zweikomponentenzusammensetzung ist, die durch Mischen einer festen ersten Komponente, die mindestens ein Peroxysalz und das Alkalisierungsmittel umfasst, und einer wässrigen zweiten Komponente, die Wasserstoffperoxid umfasst, gebildet wird.

5. Verfahren gemäß Anspruch 4, bei dem die feste erste Komponente ein Ammoniumsalz umfasst.

6. Verfahren gemäß einem der Ansprüche 1 bis 5, bei dem die Glättungszusammensetzung Glyoxylsäure und/oder ein Hydrat davon und/oder ein Salz davon in einer Konzentration im Bereich von 0,1 bis 40 Gew.%, berechnet auf Basis des Gesamtgewichts der Glättungszusammensetzung, umfasst.

7. Verfahren gemäß einem der Ansprüche 1 bis 6, **dadurch gekennzeichnet, dass** die Glättungszusammensetzung einen pH-Wert von 0,5 bis 3 aufweist.

8. Verfahren gemäß einem der Ansprüche 1 bis 7, bei dem die Glättungszusammensetzung eine oder mehrere Konditionierungskomponenten umfasst.

9. Verfahren gemäß Anspruch 8, bei dem die Konditionierungskomponente ein Silikon ist.

10. Verfahren gemäß einem der Ansprüche 1 bis 9, bei dem die Glättungszusammensetzung weniger als 2% an reduzierenden Mitteln auf Schwefelbasis umfasst.

11. Verfahren gemäß einem der Ansprüche 1 bis 10, bei dem die Glättungszusammensetzung und/oder die Bleichzusammensetzung in einem Aufbringüngsgewichtsverhältnis von Haar zur Zusammensetzung von 0,5:2 bis 2:0,5 auf das Haar aufgebracht wird.

12. Verfahren gemäß einem der Ansprüche 1 bis 11, bei dem die Temperatur des Eisens im Bereich von 170 bis 200°C liegt.

13. Verfahren gemäß einem der Ansprüche 1 bis 12, bei dem die Kombination mit dem Aufbringen einer reduzierenden Zusammensetzung oder eines alkalischen Relaxators ausgeschlossen ist.

14. Haarbehandlungskit zum Glätten und Bleichen des Haars, **dadurch gekennzeichnet, dass** es umfasst:
eine Glättungszusammensetzung, die einen pH-Wert von 4 oder niedriger aufweist und Glyoxylsäure und/oder ein Hydrat davon und/oder ein Salz davon umfasst; und
eine Bleichzusammensetzung vom Zweikomponenten-Typ, umfassend eine erste Komponente, die mindestens ein Alkalisierungsmittel umfasst, und eine zweite Komponente, die mindestens ein Oxidationsmittel umfasst, wobei die erste und die zweite Komponente dazu vorgesehen sind, vor dem Aufbringen gemischt werden, um einen pH-Wert von 8 bis 11 zu erhalten.

15. Verwendung des Haarbehandlungskits, wie in Anspruch 14 definiert, zum Glätten und Bleichen des Haars.

## Revendications

1. Procédé pour défriser et blanchir les cheveux, qui comprend les étapes suivantes réalisées dans cet ordre:
(a) appliquer sur les cheveux une première composition (composition de défrisage) ayant un pH de 4 ou moins et comprenant de l'acide glyoxylique et/ou un hydrate de celui-ci et/ou un sel de celui-ci ;
(b) laisser la composition sur les cheveux pendant 1 à 120 minutes ;
(c) sécher les cheveux,
(d) rincer éventuellement les cheveux avec de l'eau et sécher les cheveux
(e) traiter les cheveux avec un fer ayant une température de surface de 130 à 250 °C, de préférence 180 ± 50 °C,
(f) rincer éventuellement et/ou faire un shampoing et sécher les cheveux,
(g) appliquer sur les cheveux une seconde composition aqueuse (composition de blanchiment) comprenant au moins un agent oxydant et au moins un agent alcalinisant et ayant un pH de 8 à 11,
(h) laisser la composition sur les cheveux pendant 5 à 45 minutes, rincer la composition et éventuellement faire un shampoing et sécher les cheveux,
dans lequel les étapes (g) à (h) sont effectuées directement après les étapes (a) à (f).

2. Procédé selon la revendication 1, dans lequel l'agent oxydant est du peroxyde d'hydrogène, de préférence dans une proportion comprise entre 1 et 20 % en poids, par rapport au poids total de la composition de blanchiment.

3. Procédé selon l'une quelconque des revendications 1 ou 2, dans lequel l'agent alcalinisant est de l'ammoniac.

4. Procédé selon l'une quelconque des revendications 1 à 3, dans lequel la composition de blanchiment est une composition à deux composants formée en mélangeant un premier composant solide comprenant au moins un persel et l'agent alcalinisant et un second composant aqueux comprenant du peroxyde d'hydrogène.

5. Procédé selon la revendication 4, dans lequel le premier composant solide comprend un sel d'ammonium.

6. Procédé selon l'une quelconque des revendications 1 à 5, dans lequel la composition de défrisage comprend de l'acide glyoxylique et/ou un hydrate de celui-ci et/ou un sel de celui-ci à une concentration dans la plage de 0,1 à 40 % en poids, calculée sur la base du poids total de la composition de défrisage.

7. Procédé selon l'une quelconque des revendications 1 à 6, **caractérisé en ce que** la composition de défrisage a un pH de 0,5 à 3.

8. Procédé selon l'une quelconque des revendications 1 à 7, dans lequel la composition de défrisage comprend un ou plusieurs composants de conditionnement.

9. Procédé selon la revendication 8, dans lequel le composant de conditionnement est une silicone.

10. Procédé selon l'une quelconque des revendications 1 à 9, dans lequel la composition de défrisage comprend moins de 2 % d'agents réducteurs à base de soufre.

11. Procédé selon l'une quelconque des revendications 1 à 10, dans lequel la composition de défrisage et/ou la composition de blanchiment est appliquée sur les cheveux dans un rapport pondéral d'application cheveux sur composition compris entre 0,5: 2 et 2: 0,5.

12. Procédé selon l'une quelconque des revendications 1 à 11, dans lequel la température du fer est dans la plage de 170 à 200 °C.

13. Procédé selon l'une quelconque des revendications 1 à 12, dans lequel la combinaison avec l'application d'une composition réductrice ou d'un agent de relaxation alcalin est exclue.

14. Kit de traitement capillaire pour le défrisage et le blanchiment des cheveux, **caractérisé en ce qu'**il comprend:
une composition de défrisage, ayant un pH de 4 ou moins et comprenant de l'acide glyoxylique et/ou un hydrate de celui-ci et/ou un sel de celui-ci ; et
une composition de blanchiment du type à deux composants, qui comprend un premier composant comprenant au moins un agent alcalinisant, et un second composant comprenant au moins un agent oxydant, dans lequel le premier et le second composant sont fournis pour être mélangés avant l'application, pour donner un pH de 8 à 11.

15. Utilisation du kit de traitement capillaire tel que défini dans la revendication 14 pour le défrisage et le blanchiment des cheveux.
